# EUROPEAN PATENT APPLICATION

(11) **EP 4 825 558 A2**
(43) Date of publication of application: **21.10.2026**
(21) Application number: 26151790.8
(22) Date of filing: 14.01.2026
(51) Int. Cl.: B29B 17/00, G01N 33/44

(54) **METHOD FOR MANUFACTURING PLASTIC PART CONTAINING RECYCLED MATERIALS, PLASTIC PART, AND METHOD FOR IDENTIFYING RECYCLED MATERIALS IN PLASTIC PART**

(30) Priority: 14.01.2025 KR 20250005525; 11.06.2025 KR 20250076122
(71) Applicant: Hyundai Motor Company, Seoul 06797 (KR); Kia Corporation, Seoul 06797 (KR)
(72) Inventor: HONG, Chae-Hwan, 18280 Hwaseong-si, Gyeonggi-do (KR)
(74) Representative: Isarpatent

(57) **Abstract**

The present disclosure relates to a method for manufacturing a plastic part containing recycled materials, the method including steps of supplying an organic fluorescent material, mixing the organic fluorescent material with a plastic material including a recycled plastic material, melt-mixing the plastic material with the mixed organic fluorescent material, and molding a melt-mixed plastic composite, the plastic part, and a method for identifying recycled materials in the plastic part.

## Description

### BACKGROUND OF THE DISCLOSURE

### Field of the Disclosure

The present disclosure relates to a method for reliably analyzing or quantifying whether a specific material is contained or not and the content thereof by having each participant establish and comply with commitments at each stage in a supply chain involving a material manufacturer, a composite manufacturer, a part molder, and a finished vehicle manufacturer by applying a recycling or eco-friendly manufacturing method for eco-friendly plastic materials for automobiles.

### Description of Related Art

Currently, the generation of waste plastics in South Korea is increasing at an average annual rate of about 50%, but not less than 50% of the waste plastics generated are not effectively recycled and are incinerated or landfilled. However, such incineration and landfilling of waste plastics pollute water and air, causing serious environmental problems so that interest in recycling of waste plastics has been steadily increasing.

Furthermore, Europe is preparing vehicle circularity and the End-of-Life Vehicles (ELV) Regulation. This regulation regulates the adoption of circular design and the use of recycled plastic materials of a certain percentage or more. Circular design stipulates during design of new vehicles that reuse and recycling rate should be improved to a minimum of 85% and a reuse and recyclability rate should be improved to 95%. To achieve this, it mandates the design of key components with high recycling value, such as batteries, motors, engines, and gearboxes, that facilitate the disassembly of these components. Furthermore, the regulation promotes that recycled plastic materials use circular resources and mandate that at least 25% of the plastic materials used in new vehicle production consist of post-consumer recycled (PCR), of which 25% must be plastics generated from end-of-life vehicles. Therefore, if the weight of plastic used in one car is approximately 250 kg, 62.5 kg of recycled plastic is required, and approximately 15.6 kg of end-of-life vehicle-derived plastics is mixed with new plastic and applied.

Currently, some plastic waste is recycled, but most ends up in landfills, with only a small portion being used in waste incineration plants and converted into thermal energy. This approach undeniably wastes valuable raw materials that could be effectively reused and contributes to an increase in emission of greenhouse gases into the atmosphere. Recycling plastic materials particularly from the automotive industry is a critical issue for the automobile sector, which is striving for carbon neutrality. Particularly in Europe, where regulations specifying specific content levels are coming into effect, a mix of new and recycled materials is essential, and methods for identifying and quantifying these materials at each stage are needed.

When this is neglected, greenwashing (a phenomenon in which stakeholders related in the supply chain neglect the mixing management of specific materials, allowing participants at each stage to perform their roles with distorted information and ultimately convey distorted, false information to the end consumers) seriously occurs, and this will provide the government and consumer unions with a pretext for legal action, and manufacturers should face the consequences of serious brand image degradation.

Currently, plastic materials used in automobiles for interior and exterior parts and engine chassis components include polypropylene (PP), nylon, polycarbonate (PC), acrylonitrile butadiene styrene (ABS), polyethylene terephthalate (PET), etc. Among these, polypropylene is the most widely used material, followed by nylon (e.g., nylon: approximately 15 kg applied per automobile).

Therefore, technologies that reuse and recycle plastic materials used throughout the entire automobile production and scrapping process are expected to have carbon reduction effects. However, a key issue throughout this entire process is that there is a problem of distorted information being distributed, resulting in serious greenwashing in the market if there is no protocol that allows supply chain participants to trust and verify the inclusion and content of recycled materials or if such information is solely dependent on the information unilaterally provided by manufacturers at each stage, when the plastic materials are a mixture of new and recycled materials.

In other words, within the current plastic material and molding process industry structure, there is a problem in that there are no established effective methods for identifying specific materials (i.e., all methods for deriving, introducing, and analyzing specific materials) in the process of mixing new materials and specific recycled materials to newly manufacture products, i.e., more specifically, the plastic material mixing and part manufacturing steps such as plastic-applied melt extrusion, melt injection and others.

The absence of such technology causes a problem of generating intentional or unintentional market disruption, including very serious greenwashing, in the industrial environment where the utilization amount of recycled materials to be developed later is expected to increase.

The matters described in the above background art are intended to aid understanding of the background of the invention and may include matters that are not the related art already known to those skilled in the art to which the present disclosure pertains.

### SUMMARY OF THE DISCLOSURE

The present disclosure has been devised to solve the above-described problems, and an object of the present disclosure is to provide a method for quickly and highly reliably identifying whether recycled materials have been applied or not and contents thereof under conditions where new and recycled materials are mixed and applied in plastic materials for automobiles, a method for manufacturing a part that enables this, and the part.

A method for manufacturing a plastic part containing recycled materials according to one aspect of the present disclosure includes steps of supplying an organic fluorescent material, mixing the organic fluorescent material with a plastic material including a recycled plastic material, melt-mixing the plastic material with the mixed organic fluorescent material, and molding a melt-mixed plastic composite.

The plastic material is one or more selected from the group consisting of polypropylene, a polypropylene talc composite (PPtalc Composite), nylon 6, a nylon 6 glass fiber composite (Nylon 6 Glass Fiber Composite), nylon 66, a nylon 66 glass fiber composite (Nylon 66 Glass Fiber Composite), acrylonitrile butadiene styrene (ABS), and polycarbonate (PC).

In addition, the organic fluorescent material is one or more selected from stilbene and a stilbene derivative.

Here, the stilbene derivative includes 4,4-bis(2-benzoxazolyl) stilbene, N,N-bis(2-hydroxyethyl)amino-4'-cyanostilbene, N,N-bis(2-hydroxyethyl) amino-4'-methoxy stilbene, N,N-bis(2-hydroxyethyl)amino-4'-acetylstilbene, N,N-bis (2-hydroxyethyl) amino stilbene, and N,N-bis(2-hydroxyethyl)amino-4'-nitrostilbene.

Furthermore, the organic fluorescent material is added in an amount of 0.1 to 10% by weight based on 100% by weight of the plastic material.

Meanwhile, the melt-mixing step includes performing melt-mixing under a temperature of 200 to 370°C for a residence time in the equipment of 5 to 60 seconds.

The present disclosure provides a plastic part in which an organic fluorescent material is mixed with a plastic material including a recycled plastic material in an amount of 0.1 to 10% by weight based on 100% by weight of the plastic material.

Next, a method for identifying recycled materials in a plastic part according to one aspect of the present disclosure includes steps of irradiating a plastic composite in which an organic fluorescent material is mixed with a plastic material including a recycled plastic material with light having a wavelength of 190 to 900 nm, measuring fluorescence emitted from the plastic composite, and analyzing the intensity by wavelength of the measured fluorescence.

In addition, the step of analyzing the intensity includes deriving the peak intensity of the measured fluorescence and calculating the content of the organic fluorescent material from the peak intensity.

Furthermore, the step of analyzing the intensity includes calculating the content of the recycled plastic from the content of the organic fluorescent material.

The present disclosure having the above configuration has the following effects.

First, the present disclosure offers significantly higher price competitiveness compared to the previously described method for plastic materials for automobiles. This is because, in the melting and extrusion process of a specific organic fluorescent material and plastics, high fluorescence characteristics can be induced even with very small input amounts of the specific organic material.

Second, the above-described specific fluorescent material applied to plastics for automobiles exhibits no degradation in physical properties of the mechanical and durability aspects of the plastic composite molded article finally applied in the automobiles, and this provides significantly higher reliability compared to other technologies.

Third, the identification analysis protocol of the present disclosure has very important and additional advantages of providing participants participating in the supply chain with a remarkably fast analysis method and calculation method, thereby providing a platform capable of evaluating and sharing data individually among participants with high reliability.

In order to explain the features of this technology in detail, as a comparative example, one of the conventional identification methods can be considered, which is a method of mixing a solid metal particle magnetic substance into plastics to manufacture a composite, and then identifying it through a magnetic separation step and device. First, this method has the disadvantages of very low identification reliability and a large deviation in reliability depending on the surrounding environment, and also requires expensive equipment in the magnetic separation step, and when recycling the composite, it has a disadvantage in that environmental pollution occurs in the process of removing and recovering magnetic metal particles.

### BRIEF DESCRIPTION OF THE DRAWINGS

FIG. 1 illustrates a method for identifying recycled materials in plastic parts containing recycled materials according to the present disclosure.
FIG. 2 shows results of evaluating polypropylene + 4,4-bis(2-benzoxazolyl) stilbene.
FIG. 3 shows results of evaluating polypropylene talc composite + 4,4-bis(2-benzoxazolyl) stilbene.
FIG. 4 shows results of evaluating nylon 66 glass fiber composite + 4,4-bis(2-benzoxazolyl) stilbene.
FIG. 5 shows, as comparative example evaluation results, evaluation results of parts in which all types of plastic + magnetic material are iron manganese.

### DESCRIPTION OF SPECIFIC EMBODIMENTS

In order to sufficiently understand the present disclosure and operational advantages of the present disclosure, and the objects achieved by implementation of the present disclosure, reference should be made to the accompanying drawings, which illustrate preferred embodiments of the present disclosure, and the contents described in the accompanying drawings.

In describing a preferred embodiment of the present disclosure, well-known techniques or repetitive descriptions that may unnecessarily obscure the gist of the present disclosure will reduce or omit the descriptions.

The present disclosure relates to a method for reliably analyzing or quantifying whether a specific material is contained or not and the content thereof by having each participant establish and comply with commitments at each step in a supply chain involving a material manufacturer, a composite manufacturer, a part molder, and a finished vehicle manufacturer by applying a recycling or eco-friendly manufacturing method for eco-friendly plastic materials for automobiles.

Specifically, the present disclosure relates to a method for providing an active verification method and analysis method to early and intermediate stage participants or final consumers through a method of injecting a specific organic material that can include specific information (specifically, as an optical matter, emission characteristics at a specific wavelength after absorbing external energy at a specific wavelength) in a material manufacturing step, a composite manufacturing step, a part molding process step, and a finished vehicle manufacturing supply step, and more specifically, to a method for identifying and quantifying the material using a specific fluorescence analysis method and device, characterized in that the specific fluorescent material is physically dispersed or coated in a plastic material by injecting and mixing the organic fluorescent material in a specific method and content into the plastic material.

Through this, the present disclosure aims to present a method for preemptively solving potential market disruption problems, such as greenwashing, in the process of mixing new and recycled materials in plastics for automobiles.

Specifically, the present disclosure newly presents a method of deriving a specific fluorescent material that is highly compatible with plastic materials for automobiles, undergoes very little structural deformation in the melt processing process carried out under high temperature conditions, and has no inherent photochemical property modification, among over 1,000 organic materials that exhibit fluorescent properties in the process of being excited after absorbing energy from the outside and mixing this material with plastics for automobiles, and thereafter, the present disclosure presents an analysis method that can identify whether there is a specific plastic material or not and, further, the content thereof by utilizing the principle of the luminescence phenomenon, that is an optical phenomenon, (photoluminescence, a phenomenon of light emission (luminescence) without heat).

The present disclosure is characterized in that each issue of material derivation, melt mixing, and fluorescence analysis has its own features and difficulty level. Ultimately, the present disclosure designs and presents a protocol that comprehensively connects these elements, and as a result, the present disclosure presents a unique and independent identification method when recycled materials are applied, for all types of plastic materials applied in automobiles. Furthermore, the present disclosure particularly presents a fluorescent material and its identification method, which are applicable regardless of the type of plastic materials for automobiles.

More specifically, the present disclosure is for providing a method which analyzes the fluorescence characteristics of the fluorescent material using fluorescence spectroscopy by mixing a specific fluorescent material derived from the present disclosure with a plastic material or can accurately and quickly obtain specific information on recycled materials from the spectral characteristics obtained from the fluorescence spectrum. That is, when a composite is manufactured by adding a specific fluorescent material to a mixed material of a specific plastic, the fluorescent characteristics become different compared to a composite plastic to which the fluorescent material has not been added. In addition, a method which can specify the content thereof by calculating the ratio of a specific wavelength of the composite is established, and this is presented in a scientific and engineering manner.

Hereinafter, with reference to the attached drawings, a method for manufacturing a plastic part containing recycled materials according to the present disclosure, a plastic part, and a method for identifying recycled materials within the plastic part will be described.

The method includes a step (S10) of supplying an organic fluorescent material and a step (S20) of mixing the fluorescent material with plastic materials, and the plastic materials include new and recycled plastic materials. This is performed by devices storing and supplying specific fluorescent materials and devices storing and supplying specific plastics, respectively.

Then, the fluorescent material mixed with plastic materials, including new and recycled plastic materials, is melt-mixed and extruded using a composite compounding device (S30).

Next, a plastic specimen molded article is manufactured through an injection molding process using a composite manufactured from a plastic composite compounding device (S40).

Thereafter, the fluorescent material in the manufactured plastic molded article is identified (S50) by a fluorescence analysis device according to the fluorescent material detection identification protocol, and analyzed, and the results are quantified using a data processing method using the fluorescence intensity of a specific wavelength.

The present disclosure, as described above, has a special advantage that the analysis method provides very fast and highly reliable results without causing changes in the mechanical and thermal physical properties of the composite compared to, for example, a method of detecting whether metal particles utilizing magnetic force properties are present or not by adding metal particles to a plastic mixture, a detection method of adding specific inorganic particles, burning the composite, and analyzing and utilizing the properties of the inorganic particles, or the like.

In other words, since the aforementioned methods of adding metal particles and specific inorganic particles involve the inherent problem of severely degrading the fundamental mechanical and durability properties required for plastic materials for automobiles, they have a disadvantage of making it fundamentally very difficult to apply them in the automotive plastics industry. Ignoring the physical property deterioration of these mechanical and durability aspects and suggesting a method in which the metal particles and inorganic particles are detected simply by injection has no industrial validity.

More specifically, looking at the configuration, optimal content range, and upper and lower limit values of the present disclosure, the fluorescent material supply device may be a cone-style shaped device which is filled with nitrogen gas and in which a material transport screw inside is mounted.

In addition, the plastic material may be polypropylene, polypropylene talc composite (English Name: PPtalc Composite), nylon 6, nylon 6 glass fiber composite (English Name: Nylon 6 Glass Fiber Composite), nylon 66, nylon 66 glass fiber composite (English Name: Nylon 66 Glass Fiber Composite), acrylonitrile butadiene styrene (English Name: ABS), or polycarbonate (English Name: PC).

Identification of fluorescent material (S50) includes analyzing the composite specimen or plastic molded article with a fluorescence spectrometer using a general identification device to detect a fluorescent material, irradiating (inputting) light energy in a specific wavelength range, detecting the subsequent fluorescent emission in a specific wavelength range, converting it into a number, and calculating the ratio by specific wavelength using the number thus converted to calculate the content of a specific plastic.

In particular, a fluorescence spectrometer device for identifying a specific fluorescent material contained in plastics may be included. Furthermore, plastics that have passed through a melt extrusion device may be post-processed according to their intended use by a post-processing device. In addition, the identification device may include a device for supplying a specific fluorescent material added to the plastics before the plastics are fed into the melt extrusion device.

Here, the specific fluorescent material may be at least one selected from materials including stilbene and its derivative as follows.

Specifically, the stilbene derivative is preferably a monomer molecule such as 4,4-bis(2-benzoxazolyl) stilbene, N,N-bis(2-hydroxyethyl)amino-4'-cyanostilbene, N,N-bis(2-hydroxyethyl)amino-4'-methoxy stilbene, N,N-bis(2-hydroxyethyl)amino-4'-acetylstilbene, N,N-bis(2-hydroxyethyl)amino stilbene, or N,N-bis(2-hydroxyethyl)amino-4'-nitrostilbene. The characteristics of the above materials are that when an electron-withdrawing functional group is introduced as a substituent from the measurement of the absorption and fluorescence spectra of the materials, the spectrum shifts to a longer wavelength depending on the degree thereof, and conversely, when an electron-donating functional group is introduced, the spectrum shifts to a shorter wavelength.

In this embodiment, the melt extrusion device may include a pair of extrusion gears that perform melting and extrusion of the incoming plastics by being engaged with each other and rotated.

In addition, the melt extrusion device may be configured to include a feeding machine that simultaneously or sequentially supplies a certain amount of a specific fluorescent material, one type or a mixture of two or more types, to the plastics prior to performing melting and extrusion of the plastics.

In addition, the identification device can provide a device of quantifying the fluorescence emission characteristics generated after a specific fluorescent material contained in plastics absorbs energy of a specific wavelength based on the principle of fluorescence analysis method and obtaining these data, and then information that can estimate the mixing amount of recycled materials through calculation of the ratio of a specific wavelength from these data.

Specifically, the fluorescence property of a material is a phenomenon in which a specific organic molecule absorbs high-energy, short-wavelength light so that it emits light with a wavelength longer than that of absorbed light in the process of returning from an excited state to a steady state. When a molecule absorbs light, it rises to a higher-energy excited state, and if the light-absorbed molecule has risen to the excited state, the molecule becomes unstable so that it is lowered to its original state while emitting light with a different wavelength. At this time, since the energy and wavelength of light absorbed and emitted differ depending on the type of a specific fluorescent material, the analysis device uses the principle that respective fluorescent materials exhibit various fluorescent features.

The analysis device is characterized by irradiating the plastic composite with light of a wavelength ranging from ultraviolet to visible light (190 to 900 nm), and the analysis device, i.e., a fluorescence spectrophotometer, measures fluorescence emitted to measure how strong fluorescence is emitted at which wavelength. In the wavelength range of 190 nm or less to 900 nm or more, since the analytical reliability is reduced, the limitation of the above range is appropriate, and more preferably, the wavelength range between 300 nm and 600 nm is appropriate.

Such a scope of the present disclosure is not limited to the above-described embodiments, and many other variations based on the present disclosure are possible for those skilled in the art within the above-described technical scope.

In addition, the content of the specific fluorescent material to be added is preferably 0.1 to 10% by weight based on 100% by weight of the plastic material, and more preferably 0.3 to 5% by weight is appropriate. When it is less than 0.1% by weight, there is a problem in that the analysis sensitivity of the fluorescence spectrum is reduced, and when it exceeds 10% by weight, there is a disadvantage in that the discrimination of the fluorescence spectrum is reduced due to the excessive content.

In the present disclosure, the content of the specific fluorescent material depends on the content of recycled plastic. That is, if analyzing the fluorescent material depending on the content of recycled plastic, the content can be inferred depending on the ratio of its wavelengths, and a relationship graph can be obtained in advance depending on the content.

Hereinafter, the present disclosure will be described in more detail through specific Examples of the present disclosure. The following Examples are merely examples to aid understanding of the present disclosure and the scope of the present disclosure is not limited thereto.

First, Examples 1-4 were synthesized in the following manner using the components and contents shown in Table 1 blow.

### Examples 1 to 4

A feeding machine storing a particulate chemical material, 4,4-bis(2-benzoxazolyl) stilbene, a feeding machine storing materials including pelletized materials of specific plastics for automobiles (a single type or mixed type of plastics including polypropylene, pure resin, and composites reinforced with talc and glass fiber) or powdered materials obtained by being ground in advance under ultra-low temperature conditions, and a melt extrusion machine connected to a material transporting and feeding machine, which is operated at a specific rate (g/min) in each of the two feeding machines, melt-mixed a fluorescent material and a plastic resin under conditions of a temperature of 200 to 370°C and a residence time of the molten resin of 5 to 60 sec, thereby manufacturing a composite in which the fluorescent material was dispersed within the plastics.

Thereafter, fluorescence analysis was performed using a fluorescence photometer (Product Model Name: Fluorescence Spectrometer FLS980) from Edinburgh Instrument, UK. The above equipment is a device for raising a fluorescent material to a high-energy excited state, and the operating conditions thereof include application of a xenon arc lamp (lamp: Xenon Arc (150 W)), and the wavelength range of light incident on the specimen of 200 to 900 nm. The wavelength range of the fluorescence spectrum that occurs thereafter is 300 to 600 nm. The analytical equipment and specimen mounting methods, which are important configuration requirements of the present disclosure, are as follows.

Subsequently, Examples 1 to 4 were prepared in the following manner using the ingredients and contents shown in Tables 1 to 7 below, and whether the method presented in the present disclosure could identify the recycled materials or not was evaluated.

### Comparative Examples 1 to 4

The Comparative Examples were conducted using a different method from Examples 1 to 4 above, and whether the desired identification could be possible or not was evaluated.

**[Table 1]**

| Classification | Example | | | | Comparative Example | | | |
|---|---|---|---|---|---|---|---|---|
| | 1 | 2 | 3 | 4 | 1 | 2 | 3 | 4 |
| (A) Polypropylene | 100% | 90% | 80% | 70% | 100% | 90% | 80% | 70% |
| (A)-1 Recycled polypropylene | - | 10% | 20% | 30% | - | 10% | 20% | 30% |
| (B) Particulate chemical | 0.1% | 0.5% | 1% | 2% | - | - | - | - |
| material, 4,4-bis(2-benzoxazolyl) stilbene | | | | | | | | |
| (C) Magnetic nanocomposite particles characterized in that the magnetic material is manganese iron | - | - | - | - | 1% | 3% | 5% | 7% |
| (D) Melt extrusion equipment temperature (°C) | 200 | 210 | 220 | 230 | 200 | 210 | 220 | 230 |
| (E) Residence time in equipment during melt extrusion (sec) | 5 | 10 | 15 | 20 | 5 | 10 | 15 | 20 |
| (F) | O | O | O | O | X | X | X | X |
| Whether it is identifiable or not | | | | | | | | |
| - (A) Polypropylene: Reagent sold by Sigma-Aldrich, USA | | | | | | | | |
| (A)-1 Recycled polypropylene | | | | | | | | |
| - (B) 4,4-bis(2-benzoxazolyl) stilbene: Reagent sold by Sigma-Aldrich, USA | | | | | | | | |
| - (C) Magnetic nanocomposite particles characterized in that the magnetic material is manganese iron: Magnetic nanocomposite characterized by including a lactide-glycolide copolymer (the lactide-glycolide copolymer is characterized in that it has a weight average molecular weight of 5,000 to 20,000, and is a particulate material manufactured by mixing a polymer material and manganese iron particles at a weight ratio of 5 to 50) | | | | | | | | |
| - Melt extrusion equipment: Twin-screw extruder sold by Leistritz, Germany (Model name: ZSE18MAXX, screw diameter: 18.5 mm, L/D (length/diameter ratio): 60) | | | | | | | | |
| - Fluorescence analysis equipment: Equipment sold by Edinburgh Instrument, UK (Product Model Name: Fluorescence Spectrometer FLS980) | | | | | | | | |

**[Table 2]**

| Classification | Example | | | | Comparative Example | | | |
|---|---|---|---|---|---|---|---|---|
| | 1 | 2 | 3 | 4 | 1 | 2 | 3 | 4 |
| (A) Polypropylene talc composite | 100% | 90% | 80% | 70% | 100% | 90% | 80% | 70% |
| (A)-1 Recycled polypropylene talc composite | - | 10% | 20% | 30% | - | 10% | 20% | 30% |
| (B) | 0.1% | 0.5% | 1% | 2% | - | - | - | - |
| Particulate chemical material, 4,4-bis(2-benzoxazolyl) stilbene | | | | | | | | |
| (C) Magnetic nanocomposite particles characterized in that the magnetic material is manganese iron | - | - | - | - | 1% | 3% | 5% | 7% |
| (D) Melt extrusion equipment temperature (°C) | 200 | 210 | 220 | 230 | 200 | 210 | 220 | 230 |
| (E) Residence time in equipment during melt extrusion (sec) | 5 | 10 | 15 | 20 | 5 | 10 | 15 | 20 |
| (F) Whether it is identifiable or not | O | O | O | O | X | X | X | X |
| - (A) Polypropylene talc composite: Industrial resin sold by HDC Hyundai EP Co., Ltd. Korea | | | | | | | | |
| (A)-1 Recycled polypropylene talc composite | | | | | | | | |
| - (B) 4,4-bis(2-benzoxazolyl) stilbene: Reagent sold by Sigma-Aldrich, USA | | | | | | | | |
| - (C) Magnetic nanocomposite particles characterized in that the magnetic material is manganese iron: Magnetic nanocomposite characterized by including a lactide-glycolide copolymer (the lactide-glycolide copolymer is characterized in that it has a weight average molecular weight of 5,000 to 20,000, and is a particulate material manufactured by mixing a polymer material and manganese iron particles at a weight ratio of 5 to 50) | | | | | | | | |
| - Melt extrusion equipment: Commercial twin-screw extruder sold by Leistritz, Germany (Model name: ZSE18MAXX, screw diameter: 18.5 mm, L/D (length/diameter ratio): 60) | | | | | | | | |
| - Fluorescence analysis equipment: Equipment sold by Edinburgh Instrument, UK (Product Model Name: Fluorescence Spectrometer FLS980) | | | | | | | | |

**[Table 3]**

| Classification | Example | | | | Comparative Example | | | |
|---|---|---|---|---|---|---|---|---|
| | 1 | 2 | 3 | 4 | 1 | 2 | 3 | 4 |
| (A) Nylon 6 | 100% | 90% | 80% | 70% | 100% | 90% | 80% | 70% |
| (A)-1 Recycled nylon 6 | - | 10% | 20% | 30% | - | 10% | 20% | 30% |
| (B) Particulate chemical material, 4,4- | 0.1% | 0.5% | 1% | 2% | - | - | - | - |
| bis(2-benzoxazolyl) stilbene | | | | | | | | |
| (C) Magnetic nanocomposite particles characterized in that the magnetic material is manganese iron | - | - | - | - | 1% | 3% | 5% | 7% |
| (D) Melt extrusion equipment temperature (°C) | 260 | 265 | 260 | 265 | 260 | 265 | 260 | 265 |
| (E) Residence time in equipment during melt extrusion (sec) | 5 | 10 | 15 | 20 | 5 | 10 | 15 | 20 |
| (F) | O | O | O | O | X | X | X | X |
| Whether it is identifiable or not | | | | | | | | |
| - (A) Nylon 6: Industrial resin sold by HDC Hyundai EP Co., Ltd. Korea | | | | | | | | |
| (A)-1 Recycled nylon 6: Industrial resin sold by HDC Hyundai EP Co., Ltd. Korea | | | | | | | | |
| - (B) 4,4-bis(2-benzoxazolyl) stilbene: Reagent sold by Sigma-Aldrich, USA | | | | | | | | |
| - (C) Magnetic nanocomposite particles characterized in that the magnetic material is manganese iron: Magnetic nanocomposite characterized by including a lactide-glycolide copolymer (the lactide-glycolide copolymer is characterized in that it has a weight average molecular weight of 5,000 to 20,000, and is a particulate material manufactured by mixing a polymer material and manganese iron particles at a weight ratio of 5 to 50) | | | | | | | | |
| - Melt extrusion equipment: Commercial twin-screw extruder sold by Leistritz, Germany (Model name: ZSE18MAXX, screw diameter: 18.5 mm, L/D (length/diameter ratio): 60) | | | | | | | | |
| - Fluorescence analysis equipment: Equipment sold by Edinburgh Instrument, UK (Product Model Name: Fluorescence Spectrometer FLS980) | | | | | | | | |

**[Table 4]**

| Classification | Example | | | | Comparative Example | | | |
|---|---|---|---|---|---|---|---|---|
| | 1 | 2 | 3 | 4 | 1 | 2 | 3 | 4 |
| (A) Nylon 6 glass fiber composite | 100% | 90% | 80% | 70% | 100% | 90% | 80% | 70% |
| (A)-1 Recycled nylon 6 glass fiber composite | - | 10% | 20% | 30% | - | 10% | 20% | 30% |
| (B) | 0.1% | 0.5% | 1% | 2% | - | - | - | - |
| Particulate chemical material, 4,4-bis(2-benzoxazolyl) stilbene | | | | | | | | |
| (C) Magnetic nanocomposite particles characterized in that the magnetic material is manganese iron | - | - | - | - | 1% | 3% | 5% | 7% |
| (D) Melt extrusion equipment temperature (°C) | 260 | 265 | 260 | 265 | 260 | 265 | 260 | 265 |
| (E) Residence time in equipment during melt extrusion (sec) | 5 | 10 | 15 | 20 | 5 | 10 | 15 | 20 |
| (F) Whether it is identifiable or not | O | O | O | O | X | X | X | X |
| - (A) Nylon 6 glass fiber composite: Industrial resin sold by HDC Hyundai EP Co., Ltd. Korea | | | | | | | | |
| (A)-1 Recycled nylon 6 glass fiber composite: Resin obtained from HDC Hyundai EP Co., Ltd., Korea | | | | | | | | |
| - (B) 4,4-bis(2-benzoxazolyl) stilbene: Reagent sold by Sigma-Aldrich, USA | | | | | | | | |
| - (C) Magnetic nanocomposite particles characterized in that the magnetic material is manganese iron: Magnetic nanocomposite characterized by including a lactide-glycolide copolymer (the lactide-glycolide copolymer is characterized in that it has a weight average molecular weight of 5,000 to 20,000, and is a particulate material manufactured by mixing a polymer material and manganese iron particles at a weight ratio of 5 to 50) | | | | | | | | |
| - Melt extrusion equipment: Commercial twin-screw extruder sold by Leistritz, Germany (Model name: ZSE18MAXX, screw diameter: 18.5 mm, L/D (length/diameter ratio): 60) | | | | | | | | |
| - Fluorescence analysis equipment: Equipment sold by Edinburgh Instrument, UK (Product Model Name: Fluorescence Spectrometer FLS980) | | | | | | | | |

**[Table 5]**

| Classification | Example | | | | Comparative Example | | | |
|---|---|---|---|---|---|---|---|---|
| | 1 | 2 | 3 | 4 | 1 | 2 | 3 | 4 |
| (A) Nylon 66 | 100% | 90% | 80% | 70% | 100% | 90% | 80% | 70% |
| (A)-1 Recycled nylon 66 | - | 10% | 20% | 30% | - | 10% | 20% | 30% |
| (B) Particulate chemical material, 4,4- | 0.1% | 0.5% | 1% | 2% | - | - | - | - |
| bis(2-benzoxazolyl) stilbene | | | | | | | | |
| (C) Magnetic nanocomposite particles characterized in that the magnetic material is manganese iron | - | - | - | - | 1% | 3% | 5% | 7% |
| (D) Melt extrusion equipment temperature (°C) | 280 | 285 | 280 | 285 | 280 | 285 | 280 | 285 |
| (E) Residence time in equipment during melt extrusion (sec) | 5 | 10 | 15 | 20 | 5 | 10 | 15 | 20 |
| (F) | O | O | O | O | X | X | X | X |
| Whether it is identifiable or not | | | | | | | | |
| - (A) Nylon 66: Industrial resin sold by HDC Hyundai EP Co., Ltd. in Korea | | | | | | | | |
| (A)-1 Recycled nylon 66: Resin obtained from HDC Hyundai EP Co., Ltd., Korea | | | | | | | | |
| - (B) 4,4-bis(2-benzoxazolyl) stilbene: Reagent sold by Sigma-Aldrich, USA | | | | | | | | |
| - (C) Magnetic nanocomposite particles characterized in that the magnetic material is manganese iron: Magnetic nanocomposite characterized by including a lactide-glycolide copolymer (the lactide-glycolide copolymer is characterized in that it has a weight average molecular weight of 5,000 to 20,000, and is a particulate material manufactured by mixing a polymer material and manganese iron particles at a weight ratio of 5 to 50) | | | | | | | | |
| - Melt extrusion equipment: Commercial twin-screw extruder sold by Leistritz, Germany (Model name: ZSE18MAXX, screw diameter: 18.5 mm, L/D (length/diameter ratio): 60) | | | | | | | | |
| - Fluorescence analysis equipment: Equipment sold by Edinburgh Instrument, UK (Product Model Name: Fluorescence Spectrometer FLS980) | | | | | | | | |

**[Table 6]**

| Classification | Example | | | | Comparative Example | | | |
|---|---|---|---|---|---|---|---|---|
| | 1 | 2 | 3 | 4 | 1 | 2 | 3 | 4 |
| (A) Nylon 66 glass fiber composite | 100% | 90% | 80% | 70% | 100% | 90% | 80% | 70% |
| (A)-1 Recycled nylon 66 glass fiber composite | - | 10% | 20% | 30% | - | 10% | 20% | 30% |
| (B) | 0.1% | 0.5% | 1% | 2% | - | - | - | - |
| Particulate chemical material, 4,4-bis(2-benzoxazolyl) stilbene | | | | | | | | |
| (C) Magnetic nanocomposite particles characterized in that the magnetic material is manganese iron | - | - | - | - | 1% | 3% | 5% | 7% |
| (D) Melt extrusion equipment temperature (°C) | 280 | 285 | 280 | 285 | 280 | 285 | 280 | 285 |
| (E) Residence time in equipment during melt extrusion (sec) | 5 | 10 | 15 | 20 | 5 | 10 | 15 | 20 |
| (F) Whether it is identifiable or not | O | O | O | O | X | X | X | X |
| - (A) Nylon 66 glass fiber composite: Industrial resin sold by HDC Hyundai EP Co., Ltd. in Korea | | | | | | | | |
| (A)-1 Recycled nylon 66 glass fiber composite: Resin obtained from HDC Hyundai EP Co., Ltd., Korea | | | | | | | | |
| - (B) 4,4-bis(2-benzoxazolyl) stilbene: Reagent sold by Sigma-Aldrich, USA | | | | | | | | |
| - (C) Magnetic nanocomposite particles characterized in that the magnetic material is manganese iron: Magnetic nanocomposite characterized by including a lactide-glycolide copolymer (the lactide-glycolide copolymer is characterized in that it has a weight average molecular weight of 5,000 to 20,000, and is a particulate material manufactured by mixing a polymer material and manganese iron particles at a weight ratio of 5 to 50) | | | | | | | | |
| - Melt extrusion equipment: Commercial twin-screw extruder sold by Leistritz, Germany (Model name: ZSE18MAXX, screw diameter: 18.5 mm, L/D (length/diameter ratio): 60) | | | | | | | | |
| - Fluorescence analysis equipment: Equipment sold by Edinburgh Instrument, UK (Product Model Name: Fluorescence Spectrometer FLS980) | | | | | | | | |

**[Table 7]**

| Classification | Example | | | | Comparative Example | | | |
|---|---|---|---|---|---|---|---|---|
| | 1 | 2 | 3 | 4 | 1 | 2 | 3 | 4 |
| (A) Polycarbonate | 100% | 90% | 80% | 70% | 100% | 90% | 80% | 70% |
| (A)-1 Recycled polycarbonate | - | 10% | 20% | 30% | - | 10% | 20% | 30% |
| (B) Particulate chemical | 0.1% | 0.5% | 1% | 2% | - | - | - | - |
| material, 4,4-bis(2-benzoxazolyl) stilbene | | | | | | | | |
| (C) Magnetic nanocomposite particles characterized in that the magnetic material is manganese iron | - | - | - | - | 1% | 3% | 5% | 7% |
| (D) Melt extrusion equipment temperature (°C) | 290 | 295 | 290 | 295 | 290 | 295 | 290 | 295 |
| (E) Residence time in equipment during melt extrusion (sec) | 5 | 10 | 15 | 20 | 5 | 10 | 15 | 20 |
| (F) | O | O | O | O | X | X | X | X |

| Whether it is identifiable or not | | | | | | | | |
|---|---|---|---|---|---|---|---|---|
| - (A) Polycarbonate: Industrial resin sold by SABIC, Inc., USA | | | | | | | | |
| (A)-1 Recycled polycarbonate: Resin obtained from Samyang Corporation, Korea | | | | | | | | |
| - (B) 4,4-bis(2-benzoxazolyl) stilbene: Reagent sold by Sigma-Aldrich, USA | | | | | | | | |
| - (C) Magnetic nanocomposite particles characterized in that the magnetic material is manganese iron: Magnetic nanocomposite characterized by including a lactide-glycolide copolymer (the lactide-glycolide copolymer is characterized in that it has a weight average molecular weight of 5,000 to 20,000, and is a particulate material manufactured by mixing a polymer material and manganese iron particles at a weight ratio of 5 to 50) | | | | | | | | |
| - Melt extrusion equipment: Commercial twin-screw extruder sold by Leistritz, Germany (Model name: ZSE18MAXX, screw diameter: 18.5 mm, L/D (length/diameter ratio): 60) | | | | | | | | |
| - Fluorescence analysis equipment: Equipment sold by Edinburgh Instrument, UK (Product Model Name: Fluorescence Spectrometer FLS980) | | | | | | | | |

**[Table 8]**

| Classification | Example | | | | Comparative Example | | | |
|---|---|---|---|---|---|---|---|---|
| | 1 | 2 | 3 | 4 | 1 | 2 | 3 | 4 |
| (A) Acrylonitrile butadiene styrene | 100% | 90% | 80% | 70% | 100% | 90% | 80% | 70% |
| (A)-1 Recycled acrylonitrile butadiene styrene | - | 10% | 20% | 30% | - | 10% | 20% | 30% |
| (B) Particulate chemical material, 4,4-bis(2-benzoxazolyl) stilbene | 0.1% | 0.5% | 1% | 2% | - | - | - | - |
| (C) Example Results: Nylon 66 Glass Fiber Composite + 4,4-bis(2-benzoxazolyl) stilbene (x-axis: Wavelength range (300 to 600 nm) of fluorescence emitted from the specimen) (y-axis: Fluorescence | - | - | - | - | 1% | 3% | 5% | 7% |
| intensity by wavelength) | | | | | | | | |
| (D) Melt extrusion equipment temperature (°C) | 225 | 230 | 225 | 230 | 225 | 230 | 225 | 230 |
| (E) Residence time in equipment during melt extrusion (sec) | 5 | 10 | 15 | 20 | 5 | 10 | 15 | 20 |
| (F) Whether it is identifiable or not | O | O | O | O | X | X | X | X |
| - (A) Acrylonitrile butadiene styrene: Industrial resin sold by Samyang Corporation, Korea | | | | | | | | |
| (A)-1 Recycled acrylonitrile butadiene styrene: Resin obtained from Samyang Corporation, Korea | | | | | | | | |
| - (B) 4,4-bis(2-benzoxazolyl) stilbene: Reagent sold by Sigma-Aldrich, USA | | | | | | | | |
| - (C) Magnetic nanocomposite particles characterized in that the magnetic material is manganese iron: Magnetic nanocomposite characterized by including a lactide-glycolide copolymer (the lactide-glycolide copolymer is characterized in that it has a weight average molecular weight of 5,000 to 20,000, and is a particulate material manufactured by mixing a polymer material and manganese iron particles at a weight ratio of 5 to 50) | | | | | | | | |
| - Melt extrusion equipment: Commercial twin-screw extruder sold by Leistritz, Germany (Model name: ZSE18MAXX, screw diameter: 18.5 mm, L/D (length/diameter ratio): 60) | | | | | | | | |
| - Fluorescence analysis equipment: Equipment sold by Edinburgh Instrument, UK (Product Model Name: Fluorescence Spectrometer FLS980) | | | | | | | | |

Whether materials according to Examples and Comparative Examples have been identified or not is measured by the following method, and the results are as follows.

### [Evaluation Method]

### (1) Fluorescence Spectrophotometer

Fluorescence is a phenomenon in which a molecule absorbs high-energy, short-wavelength light to emit light while emitting light with a wavelength longer than that. When a molecule absorbs light, the molecule rises to an excited state with a higher energy.

If a molecule that has absorbed light rises to an excited state, the molecule becomes unstable so that it falls to its original state while releasing energy in the form of heat or emitting light of a different wavelength.

Since the energy and wavelength of light absorbed and emitted vary depending on the type of material, the respective various fluorescence spectra are exhibited. In general, fluorescence spectrophotometers differ from UV-Vis spectrophotometers. Both spectrometers share a common point of exposing a material to light with a wavelength range from ultraviolet to visible light (300 to 600 nm), causing the material to absorb light in a specific wavelength range. There is a difference in that while a UV-Vis spectrophotometer operates on a principle of measuring radiation unabsorbed by a material to check how much light is absorbed at which wavelength, a fluorescence spectrophotometer operates on a principle of measuring the fluorescence emitted from a material to check how intense the fluorescence is emitted at which wavelength.

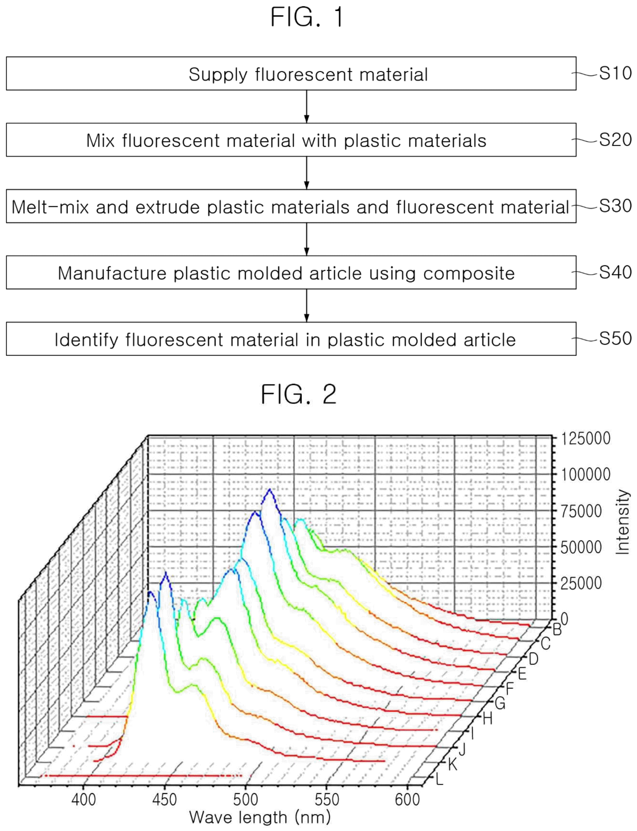

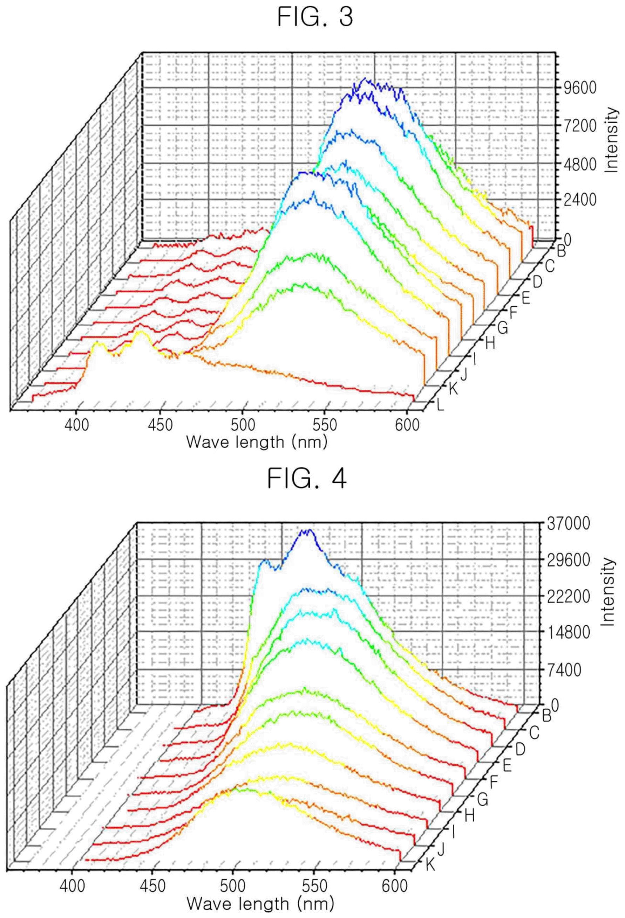

The results of analyzing the plastic phosphor composite material derived by the method derived in the present disclosure are shown in FIGS. 2 to 4. As can be seen in the result graphs of the polypropylene and polypropylene talc composite resins in FIGS. 2 and 3, the difference in fluorescence intensity at a specific wavelength is significant and is clearly observed, thereby demonstrating the success of the method of the present disclosure.

In FIGS. 2 to 5, the x-axis represents the wavelength range (300 to 600 nm) of fluorescence emitted from the specimen, and the y-axis represents the intensity of fluorescence by wavelength.

FIG. 2 shows results of evaluating polypropylene + 4,4-bis(2-benzoxazolyl) stilbene, FIG. 3 shows results of evaluating polypropylene talc composite + 4,4-bis(2-benzoxazolyl) stilbene, and FIG. 4 shows results of evaluating nylon 66 glass fiber composite + 4,4-bis(2-benzoxazolyl) stilbene.

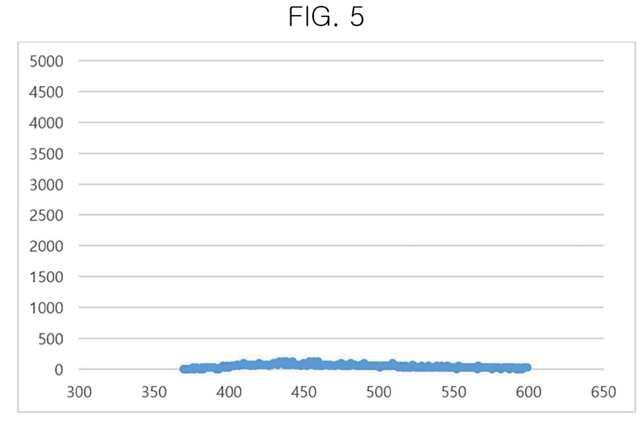

In addition, FIG. 5 is evaluation results of Comparative Examples, the Comparative Examples are magnetic nanocomposite particles characterized in that all types of plastics + magnetic material are manganese iron.

Although the present disclosure as described above has been described with reference to the illustrated drawings, it is not limited to the described embodiments, and it is obvious to those skilled in the art that various modifications and variations can be made without departing from the spirit and scope of the present disclosure. Therefore, such modified or varied examples should fall within the scope of the claims of the present disclosure, and the scope of rights of the present disclosure should be interpreted based on the appended claims.

## Claims

1. A method for manufacturing a plastic part containing recycled materials, the method comprising steps of:
supplying an organic fluorescent material;
mixing the organic fluorescent material with a plastic material including a recycled plastic material;
melt-mixing the plastic material with the mixed organic fluorescent material; and
molding a melt-mixed plastic composite.

2. The method of claim 1, wherein the plastic material is one or more selected from the group consisting of polypropylene, a polypropylene talc composite (PPtalc Composite), nylon 6, a nylon 6 glass fiber composite (Nylon 6 Glass Fiber Composite), nylon 66, a nylon 66 glass fiber composite (Nylon 66 Glass Fiber Composite), acrylonitrile butadiene styrene (ABS), and polycarbonate (PC).

3. The method of claim 1, wherein the organic fluorescent material is one or more selected from stilbene and a stilbene derivative.

4. The method of claim 3, wherein the stilbene derivative includes 4,4-bis(2-benzoxazolyl) stilbene, N,N-bis(2-hydroxyethyl)amino-4'-cyanostilbene, N,N-bis(2-hydroxyethyl) amino-4'-methoxy stilbene, N,N-bis(2-hydroxyethyl)amino-4'-acetylstilbene, N,N-bis (2-hydroxyethyl) amino stilbene, and N,N-bis(2-hydroxyethyl)amino-4'-nitrostilbene.

5. The method of claim 4, wherein the organic fluorescent material is added in an amount of 0.1 to 10% by weight based on 100% by weight of the plastic material.

6. The method of claim 1, wherein the melt-mixing step includes performing melt-mixing at a temperature of 200 to 370°C for a residence time in the equipment of 5 to 60 seconds.

7. A plastic part in which an organic fluorescent material is mixed with a plastic material including a recycled plastic material in an amount of 0.1 to 10% by weight based on 100% by weight of the plastic material.

8. The plastic part of claim 7, wherein the organic fluorescent material is one or more selected from stilbene and a stilbene derivative.

9. The plastic part of claim 8, wherein the stilbene derivative includes 4,4-bis(2-benzoxazolyl) stilbene, N,N-bis(2-hydroxyethyl)amino-4'-cyanostilbene, N,N-bis(2-hydroxyethyl) amino-4'-methoxy stilbene, N,N-bis(2-hydroxyethyl)amino-4'-acetylstilbene, N,N-bis (2-hydroxyethyl) amino stilbene, and N,N-bis(2-hydroxyethyl)amino-4'-nitrostilbene.

10. The plastic part of claim 8, wherein the plastic material is one or more selected from the group consisting of polypropylene, a polypropylene talc composite (PPtalc Composite), nylon 6, a nylon 6 glass fiber composite (Nylon 6 Glass Fiber Composite), nylon 66, a nylon 66 glass fiber composite (Nylon 66 Glass Fiber Composite), acrylonitrile butadiene styrene (ABS), and polycarbonate (PC).

11. A method for identifying recycled materials in a plastic part, the method comprising steps of:
irradiating a plastic composite in which an organic fluorescent material is mixed with a plastic material including a recycled plastic material with light having a wavelength of 190 to 900 nm;
measuring fluorescence emitted from the plastic composite; and
analyzing an intensity by wavelength of the measured fluorescence.

12. The method of claim 11, wherein the step of analyzing the intensity includes deriving a peak intensity of the measured fluorescence and calculating the content of the organic fluorescent material from the peak intensity.

13. The method of claim 12, wherein the step of analyzing the intensity includes calculating the content of the recycled plastic from the content of the organic fluorescent material.

14. The method of claim 11, wherein the organic fluorescent material is one or more selected from stilbene and a stilbene derivative.

15. The method of claim 14, wherein the stilbene derivative includes 4,4-bis(2-benzoxazolyl) stilbene, N,N-bis(2-hydroxyethyl)amino-4'-cyanostilbene, N,N-bis(2-hydroxyethyl) amino-4'-methoxy stilbene, N,N-bis(2-hydroxyethyl)amino-4'-acetylstilbene, N,N-bis (2-hydroxyethyl) amino stilbene, and N,N-bis(2-hydroxyethyl)amino-4'-nitrostilbene.
